# EUROPEAN PATENT APPLICATION

(11) **EP 2 543 382 A1**
(43) Date of publication of application: **09.01.2013**
(21) Application number: 11750801.0
(22) Date of filing: 04.03.2011
(51) Int. Cl.: A61K 38/00, A23L 1/305, A61K 35/20, A61P 21/00, A61P 21/04, A61P 43/00, A23C 21/00

(54) **AGENT FOR PREVENTING MUSCULAR ATROPHY**

(30) Priority: 04.03.2010 JP 2010048434
(71) Applicant: Megmilk Snow Brand Co., Ltd., Sapporo-shi Hokkaido 0650043 (JP)
(72) Inventor: MIURA, Susumu, Sapporo-shi Hokkaido 065-0043 (JP); NAITO, Hisashi, Chiba 270-1695 (JP)
(74) Representative: Wakerley, Helen Rachael
(86) International application number: PCT/JP2011/055037
(87) International publication number: WO 2011/108692

(57) **Abstract**

A muscle atrophy-preventing agent includes a whey protein hydrolyzate having a molecular weight distribution that is within a range of 10 kDa or less and has a main peak of 200 Da to 3 kDa, an average peptide length (APL) of 2 to 8, a free amino acid content of 20% or less, a branched-chain amino acid content of 20% or more, and an antigenicity equal to or less than 1/100,000th of that of β-lactoglobulin. The muscle atrophy-preventing agent exhibits an excellent muscle atrophy-preventing effect.

## Description

### TECHNICAL FIELD

The invention relates to a muscle atrophy-preventing agent that includes a whey protein hydrolyzate that exhibits low bitterness, excellent stability, and excellent safety as an active ingredient.
The invention also relates to a muscle atrophy-preventing food, a muscle atrophy-preventing drink, a muscle atrophy-preventing nutrient composition, and a muscle atrophy-preventing feed that include a whey protein hydrolyzate that exhibits low bitterness, excellent stability, and excellent safety as an active ingredient.
The muscle atrophy-preventing agent is effective for preventing muscle atrophy (i.e., a decrease in muscle mass) during aging, and exhibits low bitterness, excellent stability, and excellent safety.

### BACKGROUND ART

A long period of inactive or weightless state causes various physiological changes (e.g., skeletal muscle atrophy, weight loss, and bone decalcification) of a living body. For example, a long period of cast immobilization state for treating a fracture or the like, or a long period of bedridden state, causes a significant decrease in muscle mass compared with healthy individuals. It is known that an astronaut who has returned to the earth after a long stay in space cannot stand due to a decrease in muscle mass. It is also known that a decrease in muscle mass occurs during aging due to a decrease in exercise quantity or nutrition intake, so that skeletal muscle atrophy occurs. In order to deal with the above problems, attempts have been made to enable prevention of, or early recovery from, skeletal muscle atrophy using a dietary ingredient (particularly a protein and an amino acid nutrient). For example, Non-patent Document 1 discloses an effect of reducing skeletal muscle atrophy due to a long weightless state using a soybean protein as a dietary protein source, and suggests that a decrease in abdominal muscle mass is suppressed by intake of a soybean protein.
Non-patent Document 2 refers to the amount and the quality of protein that should be taken in order to prevent the progress of muscle atrophy during aging.

Cow milk or a dairy product is often considered to be the cause of a food allergy. In particular, a whey protein that is not contained in human breast milk is considered to function as an allergen. For example, Patent Document 2 discloses a method that decreases the allergenicity of a whey protein by hydrolyzing the whey protein using a protease.
It was confirmed by inhibition ELISA (see Non-Patent Document 3) that a whey protein hydrolyzate produced by the method disclosed in Patent Document 3 has an antigenicity equal to or less than 1/10,000th of that of β-lactoglobulin and a whey protein.
It was demonstrated that a whey protein hydrolyzate has a fat accumulation-inhibiting effect via oral administration. Therefore, a whey protein hydrolyzate has attracted attention as a material that has low allergenicity and high functionality.

### RELATED-ART DOCUMENT

### PATENT DOCUMENT

Patent Document 1: JP-A-2-002319
Patent Document 2: JP-A-2-138991
Patent Document 3: W02008/111562

### NON-PATENT DOCUMENT

Non-patent Document 1: Tada et al., Soy Protein Research, Vol. 2, pp. 112-117 (1999)
Non-patent Document 2: Paddon-Jones et al, Am J. Clin. Nutr., 87, pp. 1562S-6S (2008)
Non-patent Document 3: Japanese Journal of Pediatric Allergy and Clinical Immunology, 1, 36 (1987)

### SUMMARY OF THE INVENTION

### TECHNICAL PROBLEM

A soybean peptide has a muscle atrophy-preventing effect. However, since a soybean peptide has distinctive bitterness, and cloudiness occurs when dispersing a soybean peptide in water, a soybean peptide may not be used for a desired food, drink, or the like. In particular, a soybean peptide cannot be used for a product for which transparency is desired. A whey protein contained in cow milk has a high content of branched-chain amino acids (that are involved in muscle synthesis) as compared with a soybean protein, and has high net protein utilization (NPU). Therefore, it is expected that a whey protein has a muscle atrophy-preventing effect similar to or higher than that of a soybean peptide. However, the effect of intake of a whey protein or a whey peptide (i.e., whey protein hydrolyzate) on prevention of muscle atrophy has not been reported systematically. Since a whey protein hydrolyzate obtained by hydrolyzing a whey protein contained in cow milk has low antigenicity and low bitterness, and does not precipitate, a whey protein hydrolyzate may suitably be added to food or drink. However, it has not been reported that a whey protein hydrolyzate has a muscle atrophy-preventing effect.

The invention was conceived in view of the above situation. An object of the invention is to provide a muscle atrophy-preventing agent that is free from bitterness, cloudiness, and precipitation, and has a muscle atrophy-preventing effect.

### SOLUTION TO PROBLEM

The inventors of the invention conducted extensive studies, and found that a whey protein hydrolyzate obtained by hydrolyzing a whey protein contained in cow milk has a high muscle atrophy-preventing effect. This finding has led to the completion of the invention.
Specifically, the invention provides the following muscle atrophy-preventing agent, as well as the following muscle atrophy-preventing food, muscle atrophy-preventing drink, and muscle atrophy-preventing feed that include the muscle atrophy-preventing agent.

[1] A muscle atrophy-preventing agent including a whey protein hydrolyzate as an active ingredient, the whey protein hydrolyzate having (1) a molecular weight distribution that is within a range of 10 kDa or less and has a main peak of 200 Da to 3 kDa, (2) an average peptide length (APL) of 2 to 8, (3) a free amino acid content of 20% or less, (4) a branched-chain amino acid content of 20% or more, and (5) an antigenicity equal to or less than 1/100,000th of that of β-lactoglobulin.
[2] The muscle atrophy-preventing agent according to [1], wherein the whey protein hydrolyzate is obtained by performing a hydrolysis step that hydrolyzes and thermally denatures a whey protein at a pH of 6 to 10 and a temperature of 50 to 70°C using a heat-resistant protease, and an inactivation step that inactivates the protease by heating.
[3] The muscle atrophy-preventing agent according to [1], wherein the whey protein hydrolyzate is obtained by performing a preliminary hydrolysis step that hydrolyzes a whey protein at a pH of 6 to 10 and a temperature of 20 to 55°C using a protease, a hydrolysis step that hydrolyzes and thermally denatures an unhydrolyzed whey protein at a pH of 6 to 10 and a temperature of 50 to 70°C using a heat-resistant protease, and an inactivation step that inactivates the protease by heating.
[4] The muscle atrophy-preventing agent according to [1], wherein the whey protein hydrolyzate is obtained by performing a preliminary hydrolysis step that hydrolyzes a whey protein at a pH of 6 to 10 and a temperature of 20 to 55°C using a protease, a hydrolysis step that hydrolyzes and thermally denatures an unhydrolyzed whey protein at a pH of 6 to 10 and a temperature of 50 to 70°C using a heat-resistant protease, an inactivation step that inactivates the protease by heating, an ultrafiltration step that filters a reaction solution obtained by the inactivation step using an ultrafiltration membrane having a molecular weight cut-off of 1 to 20 kDa to obtain a filtrate, and a microfiltration step that filters the filtrate using a microfiltration membrane having a molecular weight cut-off of 100 to 500 kDa.
[5] A muscle atrophy-preventing food, a muscle atrophy-preventing drink, a muscle atrophy-preventing nutrient composition, or a muscle atrophy-preventing feed including the muscle atrophy-preventing agent according to any one of [1] to [4].
[6] A method of producing a muscle atrophy-preventing agent including a hydrolysis step that hydrolyzes and thermally denatures a whey protein at a pH of 6 to 10 and a temperature of 50 to 70°C using a heat-resistant protease, and an inactivation step that inactivates the protease by heating.
[7] The method according to [6], further including, before the hydrolysis step, a preliminary hydrolysis step that hydrolyzes the whey protein at a pH of 6 to 10 and a temperature of 20 to 55°C using a protease.
[8] The method according to [6] or [7], further including, after the inactivation step, an ultrafiltration step that filters a reaction solution obtained by the inactivation step using an ultrafiltration membrane having a molecular weight cut-off of 1 to 20 kDa to obtain a filtrate, and a microfiltration step that filters the filtrate using a microfiltration membrane having a molecular weight cut-off of 100 to 500 Da to obtain a retentate.
[9] A muscle atrophy-preventing method including administering a whey protein hydrolyzate in an amount of 10 g/day or more, the whey protein hydrolyzate having (1) a molecular weight distribution that is within a range of 10 kDa or less and has a main peak of 200 Da to 3 kDa, (2) an average peptide length (APL) of 2 to 8, (3) a free amino acid content of 20% or less, (4) a branched-chain amino acid content of 20% or more, and (5) an antigenicity equal to or less than 1/100,000th of that of β-lactoglobulin.

### ADVANTAGEOUS EFFECTS OF THE INVENTION

The muscle atrophy-preventing agent according to one aspect of the invention exhibits a remarkable muscle atrophy-preventing effect.

### DESCRIPTION OF EMBODIMENTS

The invention is described in detail below.

### Active ingredient

A whey protein hydrolyzate that is used as an active ingredient of a muscle atrophy-preventing agent according to one embodiment of the invention has (1) a molecular weight distribution that is within a range of 10 kDa or less and has a main peak of 200 Da to 3 kDa, (2) an average peptide length (APL) of 2 to 8, (3) a free amino acid content of 20% or less, (4) a branched-chain amino acid content of 20% or more, and (5) an antigenicity equal to or less than 1/100,000th of that of β-lactoglobulin.

Since the whey protein hydrolyzate has an antigenicity equal to or less than 1/100,000th of that of β-lactoglobulin and a whey protein, the whey protein hydrolyzate is highly safe in terms of suppressing a food allergy. An aqueous solution of the whey protein hydrolyzate is transparent, and has a bitterness of about 2. Therefore, the whey protein hydrolyzate does not impose limitations to the muscle atrophy-preventing agent in terms of flavor (taste) and appearance. In particular, a large amount of the whey protein hydrolyzate can be present even in the muscle atrophy-preventing agent for which transparency is particularly desired. The water-solubility of the whey protein hydrolyzate can be improved by filtering the whey protein hydrolyzate using an ultrafiltration (UF) membrane or a microfiltration (MF) membrane.

The muscle atrophy-preventing agent can be used as an active ingredient for a muscle atrophy-preventing food, a muscle atrophy-preventing drink, a muscle atrophy-preventing nutrient composition, or a muscle atrophy-preventing feed that has the above a muscle atrophy-preventing effect, and is very safe.

Since the muscle atrophy-preventing agent according to one embodiment of the invention is produced using a whey protein as a raw material, the muscle atrophy-preventing agent can be produced easily and economically.

### Production method

The whey protein hydrolyzate included in the muscle atrophy-preventing agent according to one embodiment of the invention is obtained by hydrolyzing and thermally denaturing a whey protein at a pH of 6 to 10 and a temperature of 50 to 70°C using a heat-resistant protease, and inactivating the protease by heating. The yield of the whey protein hydrolyzate can be improved by preliminarily hydrolyzing the whey protein at a pH of 6 to 10 and a temperature of 20 to 55°C using a protease, and then immediately hydrolyzing the whey protein under the above conditions without cooling the whey protein.

The muscle atrophy-preventing effect can be improved by concentrating the whey protein hydrolyzate obtained as described above using an ultrafiltration (UF) membrane having a molecular weight cut-off of 1 to 20 kDa (preferably 2 to 10 kDa) and/or a microfiltration (MF) membrane having a molecular weight cut-off of 100 to 500 Da (preferably 150 to 300 Da). The whey protein hydrolyzate thus obtained exhibits lower bitterness and improved transparency.

The term "whey protein" used herein refers to whey of a mammal (e.g., cow, buffalo, goat, or human), an aggregate, a powder, or a purified protein thereof. The whey protein is used in a state of an aqueous solution when hydrolyzing the whey protein using a protease.

The pH of the whey protein aqueous solution is adjusted to 6 to 10. Since the whey protein aqueous solution normally has a pH within the above range, it is normally unnecessary to adjust the pH of the whey protein aqueous solution. When it is necessary to adjust the pH of the whey protein aqueous solution, the pH of the whey protein aqueous solution is adjusted to 6 to 10 using a solution of an acid (e.g., hydrochloric acid, citric acid, or lactic acid) or an alkali (e.g., caustic soda, calcium hydroxide, or sodium phosphate). The whey protein aqueous solution is heated to 50 to 70°C. It is preferable to add the heat-resistant protease before heating the whey protein aqueous solution so that hydrolysis also occurs during heating (i.e., the yield is improved).

The optimum temperature for a normal protease is 40°C or less, and the optimum temperature for a heat-resistant protease is 45°C or more. An arbitrary heat-resistant protease may be used as long as the heat-resistant protease has an optimum temperature of 45°C or more. Examples of such a heat-resistant protease include papain, Protease S (trade name), Proleather (trade name), Thermoase (trade name), Alcalase (trade name), Protin A (trade name), and the like. It is preferable to use a heat-resistant protease that has a residual activity of 10% or more when heated at 80°C for 30 minutes. It is more effective to use a plurality of proteases in combination. The reaction time is preferably about 30 minutes to about 10 hours.

The reaction solution is then heated to inactivate the protease. The protease may be inactivated by heating the reaction solution at 100°C or more for 10 seconds or more.

After inactivating the protease, the reaction solution is centrifuged. The supernatant liquid is then collected, and dried to obtain a powdery product. Since a precipitate that occurs due to centrifugation is at a lower level of hypoallergenic property as compared with the supernatant liquid, it is preferable to remove the precipitate. Note that the reaction solution may be dried and used directly as long as there is not a problem of antigenicity.

It was confirmed that the whey protein hydrolyzate obtained by the above method has an antigenicity equal to or less than 1/100,000th of that of β-lactoglobulin and a whey protein when measured by inhibition ELISA. Therefore, the whey protein hydrolyzate is highly safe. An aqueous solution of the whey protein hydrolyzate is transparent, and has a bitterness of about 2. Therefore, the whey protein hydrolyzate rarely poses a problem in terms of flavor (taste) and appearance. Note that the transparency and the bitterness of the whey protein hydrolyzate are evaluated by the following methods.

Transparency evaluation method: A1% whey protein hydrolyzate solution is prepared, and the absorbance at 650 nm is measured.

Bitterness evaluation method: A 10% whey protein hydrolyzate solution is prepared, and the bitterness of the solution is evaluated using quinine hydrochloride (bitter substance). A whey protein hydrolyzate having a bitterness of 2 or less (see Table 1) can be used for food, drink, or the like.

The APL of the whey protein hydrolyzate may be determined by HPLC or the like.

**TABLE 1**

| Quinine hydrochloride concentration | Bitterness |
|---|---|
| 0.004% | 1 (Low) |
| 0.010% | 2 |
| 0.020% | 3 (High) |

### Usage

The whey protein hydrolyzate according to one embodiment of the invention may be used directly as a muscle atrophy-preventing agent, or may be incorporated in a powdered drug, granules, a tablet, a capsule, a drinkable preparation, or the like using a normal method. A whey protein hydrolyzate obtained using an ultrafiltration (UF) membrane or a microfiltration (MF) membrane may be used directly as a muscle atrophy-preventing agent, or may be used after drying. The whey protein hydrolyzate may also be prepared as a drug or the like using a normal method.

The whey protein hydrolyzate that has been prepared as a drug or the like may be added to a nutrient preparation, food or drink (e.g., yogurt drink, milk-based drink, or wafer), feed, a supplement, or the like.

The content of the whey protein hydrolyzate in a muscle atrophy-preventing food, a muscle atrophy-preventing drink, a muscle atrophy-preventing nutrient composition, or a muscle atrophy-preventing feed is not particularly limited, but is preferably determined so that an adult can take the whey protein hydrolyzate in an amount of 10 g/day or more, and preferably 20 g/day or more.

The whey protein hydrolyzate used as an active ingredient may be mixed with an appropriate adjuvant, and formed into an arbitrary oral preparation (muscle atrophy-preventing agent).

The muscle atrophy-preventing agent according to one embodiment of the invention may be applied to an arbitrary person, but is preferably applied to middle-aged persons (after age 30), elderly persons, persons who are in a long-term inactive state (e.g., a long-term cast immobilization state or a long-term bedridden state), and persons who stay in space.

The invention is further described below by way of examples and comparative examples. Note that the invention is not limited to the following examples.

### Example 1

Papain (50 U/g.whey protein) and Proleather (manufactured by Amano Enzyme Inc.) (150 U/g.whey protein) were added to 1 liter of a 10% whey protein aqueous solution. After adjusting the pH of the mixture to 8, the whey protein was hydrolyzed and denatured at 55°C for 6 hours. The reaction solution was heated at 100°C for 15 seconds or more to inactivate the proteases. The reaction solution was then centrifuged, and the supernatant liquid was collected, and dried to obtain a whey protein hydrolyzate (product of Example 1).

The whey protein hydrolyzate (product of Example 1) had a molecular weight distribution that was within a range of 10 kDa or less and had a main peak of 1.3 kDa, an APL of 7.2, and a free amino acid content of 18.9%.

The whey protein hydrolyzate (product of Example 1) had an antigenicity equal to or less than 1/100,000th of that of β-lactoglobulin (measured by inhibition ELISA). The yield (i.e., the ratio (%) of the dry weight of the supernatant liquid to the dry weight of the raw material) was 80.3%, and the bitterness was 2.

The whey protein hydrolyzate (product of Example 1) thus obtained can be used directly as the muscle atrophy-preventing agent according to one embodiment of the invention.

### Example 2

Papain (50 U/g.whey protein) and Proleather (150 U/g.whey protein) were added to 1 liter of a 10% whey protein aqueous solution. After adjusting the pH of the mixture to 8, the whey protein was hydrolyzed at 50°C for 3 hours. The mixture was heated to 55°C, and the whey protein was hydrolyzed and denatured at 55°C for 3 hours. Next, the mixture was heated at 100°C for 15 seconds or more to inactivate the proteases. The reaction solution was filtered through a UF membrane having a molecular weight cut-off of 10 kDa (manufactured by STC) and an MF membrane having a molecular weight cut-off of 300 Da (manufactured by STC) to collect a concentrate fraction. The fraction was dried to obtain a whey protein hydrolyzate (product of Example 2).

The whey protein hydrolyzate (product of Example 2) had a molecular weight distribution that was within a range of 10 kDa or less and had a main peak of 500 Da, an APL of 3.0, and a free amino acid content of 15.2%.

The whey protein hydrolyzate (product of Example 2) had an antigenicity equal to or less than 1/100,000th of that of β-lactoglobulin (measured by inhibition ELISA). The yield was 65.4%, and the bitterness was 2. The whey protein hydrolyzate (product of Example 2) thus obtained can be used directly as the muscle atrophy-preventing agent according to one embodiment of the invention.

### Comparative Example 1

120 g of a whey protein was dissolved in 1800 ml of purified water, and the pH of the solution was adjusted to 7.0 using a 1M caustic soda solution. The solution was sterilized at 60°C for 10 minutes, and held at 45°C. After the addition of 20 g of Amano A (manufactured by Amano Enzyme Inc.), the mixture was reacted for 2 hours. The mixture was then heated at 80°C for 10 minutes to inactivate the protease, and freeze-dried to obtained a whey protein hydrolyzate (product of Comparative Example 1). The hydrolysis rate of the whey protein hydrolyzate was 18%, and the yield was 80.6%.

### Comparative Example 2

120 g of a whey protein was dissolved in 1800 ml of purified water, and the pH of the solution was adjusted to 7.0 using a 1M caustic soda solution. The solution was sterilized at 60°C for 10 minutes, and held at 45°C. After the addition of 20 g of Amano A (manufactured by Amano Enzyme Inc.), the mixture was reacted for 8 hours. The mixture was then heated at 80°C for 10 minutes to inactivate the protease, and freeze-dried to obtained a whey protein hydrolyzate. The hydrolysis rate of the whey protein hydrolyzate was 30%, and the yield was 80.6%.

### Test Example 1

### Transparency test

A 1% aqueous solution of each of the whey protein hydrolyzates obtained in Examples 1 and 2 and Comparative Examples 1 and 2 was prepared, and the absorbance at 650 nm was measured. The results are shown in Table 2.

**TABLE 2**

| Sample | Absorbance (650nm) |
|---|---|
| Product of Example 1 | 0. 008 |
| Product of Example 2 | 0. 004 |
| Product of Comparative Example 1 | 0. 064 |
| Product of Comparative Example 2 | 0. 018 |

It was thus confirmed that the whey protein hydrolyzates obtained in Examples 1 and 2 had a low absorbance (i.e., high transparency). The whey protein hydrolyzates obtained in Comparative Examples 1 and 2 had a high absorbance (i.e., low transparency) as compared with the whey protein hydrolyzates obtained in Examples 1 and 2. The whey protein hydrolyzate obtained in Example 2 that was subjected to the membrane treatment had a low absorbance (i.e., high transparency) as compared with the whey protein hydrolyzate obtained in Example 1.

### Test Example 2

### Muscle atrophy prevention test

SAM-P female rats (20 weeks old) purchased from Japan SLC Inc. were divided into five groups so that the weight of each group was equal (n=6 to 10, 144±6.8 g).

The rats were arbitrarily fed a standard AIN93 diet or a standard AIN93 diet in which an unhydrolyzed whey protein (WPC), the whey protein hydrolyzate obtained in Example 1, the whey protein hydrolyzate obtained in Example 2, or the whey protein hydrolyzate obtained in Comparative Example 1 was used instead of a casein protein. The diet intake was measured every 2 to 3 days.

After keeping the rats of each group up to 45 weeks old, the extensor digitorum longus (EDL), the tibialis anterior (TA), the soleus muscle (SOL), and the gastrocnemius (GAS) were removed under anesthesia with pentobarbital sodium (50 mg/kg), and the muscle mass was measured. The measurement results of the relative muscle mass based on the weight of the rat are shown in Table 3.

**TABLE 3**

| Sample | EDL | TA | SOL | GAS |
|---|---|---|---|---|
| Standard diet | 0. 28±0.05 a | 0.90±0.02 b | 0.16±0.02 | 2.90±0.02 c |
| WPC | 0.24±0.05 | 0.93±0.03 b | 0.15±0.02 | 2.70±0.02 |
| Product of Example 1 | 0.31±0.05 | 1.20±0.05 | 0.18±0.02 | 3.50±0.04 |
| Product of Example 2 | 0.31±0.04 | 1.20±0.01 | 0.18±0.02 | 3.15±0.03 |
| Product of Comparative Example 1 | 0.23±0.05 | 0.94±0.03 b | 0.16±0.02 | 2.60±0.02 |

| | | | | |
|---|---|---|---|---|
| a A significant difference from the products of the examples was observed (p<0.05) b A significant difference from the products of the examples was observed (p<0 05) c A significant difference from the products of the examples was observed (p<0.05) | | | | |

The relative mass of the tibialis anterior based on the weight of the rat was significantly low in the standard diet group and the whey protein group as compared with the group that was fed the whey protein hydrolyzate obtained in Example 1 and the group that was fed the whey protein hydrolyzate obtained in Example 2. The relative mass of the extensor digitorum longus, the soleus muscle, and the gastrocnemius tended to be low in the standard diet group and the whey protein group as compared with the group that was fed the whey protein hydrolyzate obtained in Example 1 and the group that was fed the whey protein hydrolyzate obtained in Example 2. The relative muscle mass based on the weight of the rat was significantly low in the standard diet group, the whey protein group, and the group that was fed the whey protein hydrolyzate obtained in Comparative Example 1 as compared with the group that was fed the whey protein hydrolyzate obtained in Example 1 and the group that was fed the whey protein hydrolyzate obtained in Example 2. The relative mass of the extensor digitorum longus, the soleus muscle, and the gastrocnemius tended to be low in the standard diet group, the whey protein group, and the group that was fed the whey protein hydrolyzate obtained in Comparative Example 1 as compared with the group that was fed the whey protein hydrolyzate obtained in Example 1 and the group that was fed the whey protein hydrolyzate obtained in Example 2. Specifically, since the SAM-P female rat is a senescence-accelerated mouse, muscle atrophy occurred during aging, and the muscle mass decreased when feeding the standard diet. On the other hand, muscle atrophy (i.e., a decrease in muscle mass) was suppressed in the group that was fed the whey protein hydrolyzate obtained in Example 1 and the group that was fed the whey protein hydrolyzate obtained in Example 2. The above results demonstrate that the muscle atrophy-preventing agent according to one embodiment of the invention exhibits an excellent muscle atrophy-preventing effect.

### Example 3

### Production of muscle atrophy-preventing tablet

Raw materials were mixed in the ratio shown in Table 4. 1 g of the mixture was formed and tableted by a normal method to produce a muscle atrophy-preventing tablet according to one embodiment of the invention.

**TABLE 4**

| | |
|---|---|
| Hydrated crystalline glucose | 73.5 (wt%) |
| Product of Example 1 | 20.0 |
| Mineral mixture | 5.0 |
| Sugar ester | 1.0 |
| Essence | 0.5 |

### Example 4

### Production of muscle atrophy-preventing nutrient composition

500 g of the product of Example 2 was dissolved in 4500 g of deionized water. The solution was heated to 50°C, and stirred at 6000 rpm for 30 minutes using a TK-homomixer ("TK ROBO MICS" manufactured by PRIMIX Corporation) to obtain a solution A. 5.0 kg of casein, 5.0 kg of a soybean protein, 1.0 kg of fish oil, 3.0 kg of perilla oil, 18.0 kg of dextrin, 6.0 kg of a mineral mixture, 1.95 kg of a vitamin mixture, 2.0 kg of an emulsifier, 4.0 kg of a stabilizer, and 0.05 kg of essence were added to 5.0 kg of the solution A, and a retort pouch (200 ml) was charged with the mixture. The mixture was then sterilized at 121°C for 20 minutes using a retort sterilizer (class-1 pressure vessel, "RCS-4CRTGN" manufactured by Hisaka Works, Ltd.) to produce 50 kg of a muscle atrophy-preventing nutrient composition according to one embodiment of the invention.

### Example 5

### Production of muscle atrophy-preventing drink

30 g of a skimmed milk powder was dissolved in 670 g of deionized water, and 10 g of the product of Example 1 was dissolved in the solution. The resulting solution was heated to 50°C, and stirred at 9500 rpm for 30 minutes using an ultra-disperser ("ULTRA-TURRAX T-25" manufactured by IKA Japan). After the addition of 100 g of maltitol, 2 g of an acidifier, 20 g of reduced starch syrup, 2 g of essence, and 166 g of deionized water, a glass bottle (100 ml) was charged with the mixture. After sterilization at 90°C for 15 minutes, the bottle was sealed to obtain ten bottles (100 ml) of a muscle atrophy-preventing drink according to one embodiment of the invention.

## Claims

1. A muscle atrophy-preventing agent comprising a whey protein hydrolyzate as an active ingredient, the whey protein hydrolyzate having (1) a molecular weight distribution that is within a range of 10 kDa or less and has a main peak of 200 Da to 3 kDa, (2) an average peptide length (APL) of 2 to 8, (3) a free amino acid content of 20% or less, (4) a branched-chain amino acid content of 20% or more, and (5) an antigenicity equal to or less than 1/100,000th of that of β-lactoglobulin.

2. The muscle atrophy-preventing agent according to claim 1, wherein the whey protein hydrolyzate is obtained by performing a hydrolysis step that hydrolyzes and thermally denatures a whey protein at a pH of 6 to 10 and a temperature of 50 to 70°C using a heat-resistant protease, and an inactivation step that inactivates the protease by heating.

3. The muscle atrophy-preventing agent according to claim 1, wherein the whey protein hydrolyzate is obtained by performing a preliminary hydrolysis step that hydrolyzes a whey protein at a pH of 6 to 10 and a temperature of 20 to 55°C using a protease, a hydrolysis step that hydrolyzes and thermally denatures an unhydrolyzed whey protein at a pH of 6 to 10 and a temperature of 50 to 70°C using a heat-resistant protease, and an inactivation step that inactivates the protease by heating.

4. The muscle atrophy-preventing agent according to claim 1, wherein the whey protein hydrolyzate is obtained by performing a preliminary hydrolysis step that hydrolyzes a whey protein at a pH of 6 to 10 and a temperature of 20 to 55°C using a protease, a hydrolysis step that hydrolyzes and thermally denatures an unhydrolyzed whey protein at a pH of 6 to 10 and a temperature of 50 to 70°C using a heat-resistant protease, an inactivation step that inactivates the protease by heating, an ultrafiltration step that filters a reaction solution obtained by the inactivation step using an ultrafiltration membrane having a molecular weight cut-off of 1 to 20 kDa to obtain a filtrate, and a microfiltration step that filters the filtrate using a microfiltration membrane having a molecular weight cut-off of 100 to 500 Da.

5. A muscle atrophy-preventing food, a muscle atrophy-preventing drink, a muscle atrophy-preventing nutrient composition, or a muscle atrophy-preventing feed comprising the muscle atrophy-preventing agent according to any one of claims 1 to 4.

6. A method of producing a muscle atrophy-preventing agent comprising a hydrolysis step that hydrolyzes and thermally denatures a whey protein at a pH of 6 to 10 and a temperature of 50 to 70°C using a heat-resistant protease, and an inactivation step that inactivates the protease by heating.

7. The method according to claim 6, further comprising, before the hydrolysis step, a preliminary hydrolysis step that hydrolyzes the whey protein at a pH of 6 to 10 and a temperature of 20 to 55°C using a protease.

8. The method according to claim 6 or 7, further comprising, after the inactivation step, an ultrafiltration step that filters a reaction solution obtained by the inactivation step using an ultrafiltration membrane having a molecular weight cut-off of 1 to 20 kDa to obtain a filtrate, and a microfiltration step that filters the filtrate using a microfiltration membrane having a molecular weight cut-off of 100 to 500 Da to obtain a retentate.

9. A muscle atrophy-preventing method comprising administering a whey protein hydrolyzate in an amount of 10 g/day or more, the whey protein hydrolyzate having (1) a molecular weight distribution that is within a range of 10 kDa or less and has a main peak of 200 Da to 3 kDa, (2) an average peptide length (APL) of 2 to 8, (3) a free amino acid content of 20% or less, (4) a branched-chain amino acid content of 20% or more, and (5) an antigenicity equal to or less than 1/100,000th of that of β-lactoglobulin.
